# EUROPEAN PATENT APPLICATION

(11) **EP 0 726 059 A1**
(43) Date of publication of application: **14.08.1996**
(21) Application number: 95120635.8
(22) Date of filing: 28.12.1995
(51) Int. Cl.: A61B 1/05, H01R 13/719

(54) **Electronic endoscope**

(30) Priority: 08.02.1995 JP 20763/95
(71) Applicant: OLYMPUS OPTICAL CO., LTD., Tokyo 151 (JP)
(72) Inventor: Yabe, Hisao, Hachioji-shi, Tokyo (JP); Kitano, Seiji, Hachioji-shi, Tokyo (JP); Nakazawa, Masaaki, Hino-shi, Tokyo (JP)
(74) Representative: Kahler, Kurt, Dipl.-Ing.

(57) **Abstract**

A connector (1) which is connected to a light source device is provided at a distal end of a connection cord which extends from an operation part of an electronic endoscope. A connection part (93) is provided within a failure prevention (149) which is formed in the vicinity of a distal end of the connection cord to which a rearward end of the connector (1) is connected. A signal cable (97) is inserted and passes through a ferrite core (30) which is arranged within the connection part (93), and which serves as a tubular electromagnetic absorber. Fixing is made to the signal cable (97) by a heat contraction tube (161). Peripheral noises around the signal cable (97) are absorbed by the ferrite core (30).

## Description

### BACKGROUND OF THE INVENTION

### Field of the Invention and Related Art Statement

The invention relates to an electronic endoscope which is provided with an electromagnetic absorber for restraining radiation or the like of noises through a signal cable.

In an endoscope apparatus, noises which are generated from a video processor, a light source device and the like are transmitted through a signal cable which is connected to a solid-state image pickup element which is provided in a forward end of an endoscope, to exert influence upon the endoscope. In view of this, hitherto, in peripheral equipments such as the video processor, the light source device and the like, the radiative noises are reduced, and malfunction of the peripheral equipments due to the noises is prevented from being generated.

Hitherto, in the electronic endoscope, the signal cable is lengthened more than the length of the electronic endoscope, in consideration of repair. The excessive length of the signal cable is wound anywhere around anything within the electronic endoscope, and is stored. Alternatively, the excessive length is stored together.

Accordingly, when current flows through the signal cable, a magnetic field is generated, as well known, from a storage portion around which the signal cable is wound, resulting in a generation source for the radiative noises.

Conventionally, reduction countermeasures for the radiative noises have been executed by the peripheral equipments. However, if the storage part is located toward the forward end of the electronic endoscope more than the video processor, it becomes difficult to effectively prevent the noises from being radiated or from being invaded from the connector part with respect to the video processor or the like so that the invaded noises are easy to be transmitted to the forward end of the electronic endoscope.

Since the solid-state image pickup element is built in the forward end of the electronic endoscope, the noises are superimposed upon a feeble or weak video signal so that there is the possibility that this causes the image quality of the endoscope image to be reduced.

Further, a connector is provided at a distal end of a universal cord or a connection cord which extends from the electronic endoscope. A light guide fiber which transmits the illumination light has an end thereof which is provided on the connector. The end of the light guide fiber is connected to the light source device.

Further, the connector is provided with a connector receipt to which the distal end of the signal cable connected to the solid-state image pickup element is connected, and is connected to the video processor through a connection cable which is provided with a connector connected to the connector receipt.

Accordingly, in a case where the connector is connected to the light source device, the connector receipt is arranged in the vicinity of the light source device. Thus, the drive signal which drives the image pickup element, for example, is radiated toward the power supply or power source circuit within the light source device, This makes noises. Thus, there is the possibility that influence is exerted upon the peripheral equipments which form the endoscope apparatus, such as the control circuit within the light source device, or the like. Reversely, the noises from the power supply circuit or the like within the light source device become easy to be superimposed upon the signal cable. In a case where the noises are superimposed upon the signal cable, this makes noises to the weak video signal due to the solid-state image pickup element.

Moreover, if the noises are superimposed upon the signal cable, the signal cable serves as an antenna, and the noises are radiated to the circumference. Thus, there is the possibility that the noises are given to the peripheral equipments which form the endoscope apparatus.

### OBJECTS AND SUMMARY OF THE INVENTION

An object of the present invention is to provide an electronic endoscope which reduces that noises radiated through a signal cable so as not to malfunction peripheral devices of an endoscope apparatus.

The other object of the present invention is to provide an electronic endoscope which prevents noises from being invaded through a signal cable so that an image which is superior in S/N is obtained.

According to the invention, there is provided an electronic endoscope comprising:
a signal cable connected to an image pickup element, a connection cord through which a light guide fiber for transmitting an illumination light is inserted and passes, and which extends to the outside from an operation part, and
a connector provided at a distal end of said connection cord and detachably connected to a light source device,
wherein it comprises:
a connection part provided in the vicinity of the distal end of said connection cord and connected to said connector, and
an electromagnetic absorber arranged within said connection part and having function to absorb an electromagnetic wave through said signal cable. Thus, radiation of the noises through the signal cable and invasion of the noises into the signal cable are reduced, whereby malfunction of peripheral devices are prevented from occurring, and an image which is superior in S/N is obtained.

### BRIEF DESCRIPTION OF THE DRAWINGS

Figs. 1 to 19 relate to a first embodiment of the invention, Fig. 1 being an overall arrangement view of an endoscope apparatus which is provided with an electronic endoscope according to the first embodiment;
Fig. 2 is a cross-sectional view showing an arrangement which includes signal contacts in a connector;
Fig. 3 is a cross-sectional view showing an arrangement or structure which includes a light guide in the connector;
Fig. 4 is a side elevational view to partially cut out the connector, showing an arrangement of a gas-supply base portion;
Fig. 5 is a cross-sectional view showing an arrangement in the vicinity of an electric contact in the connector;
Fig. 6 is a side elevational view to cut out the connector, showing an arrangement in the vicinity of a signal cable within a connection part;
Fig. 7 is a side elevational view showing an arrangement in the vicinity of the signal cable within the connection part, similarly to Fig. 6;
Fig. 8 is a cross-sectional view showing an arrangement of a failure prevention portion of a connection cord;
Fig. 9 is a cross-sectional view showing an arrangement of the connection part;
Fig. 10 is a perspective view showing a configuration or shape of a base;
Fig. 11 is a view showing a second block with a part thereof cut out;
Fig. 12 is a view showing a configuration or shape of a shielding plate;
Figs. 13A and 13B are cross-sectional views showing an arrangement in the vicinity of an earth terminal and an arrangement in the vicinity of a gas supply base in the connector;
Figs. 14A and 14B are cross-sectional views showing the gas supply base and a gas supply port;
Fig. 15 is a view showing an inflow prevention plate;
Fig. 16 is a schematic cross-sectional view of a ferrite core under a state in which the signal cable is inserted;
Figs. 17A and 17B are schematic cross-sectional views of the ferrite core under a state in which a signal cable in a modification in Fig. 16 passes;
Fig. 18 is a schematic cross-sectional view of a further another modification in Fig. 16;
Fig. 19 is a cross-sectional view showing a fixed or stationary mechanism portion between a second body block and the connection part;
Fig. 20 is a view of the fact that the connector in a second embodiment is partially cut out to show the vicinity of the connection part;
Fig. 21 is a view the fact that the connector in a third embodiment is partially cut out to show the vicinity of the connection part;
Fig. 22 is a cross-sectional view showing the connection part which includes fixing means of a metal plate;
Fig. 23 is a view showing a form or contour of the metal plate;
Fig. 24 is a view showing the fixing means for the metal plate;
Figs. 25A and 25B are cross-sectional views showing the connection part which includes the fixing means for the metal plate;
Figs. 26A and 26B are views showing the fixing means for the metal plate and the connection part in a fourth embodiment;
Figs. 27A and 27B are views showing the fixing means for the metal plate and the connection part;
Figs. 28A and 28B are views showing the fixing means for the metal plate and the connection part;
Fig. 29 is a cross-sectional view showing the connection part which includes the fixing means for the metal plate in a fifth embodiment;
Fig. 30 is a cross-sectional view showing the connection part which includes the fixing means for the metal plate;
Fig. 31 is a cross-sectional view showing the connection part which includes the fixing means for the metal plate; and
Figs. 32A and 32B show the fixing means for the ferrite core and a cross-sectional view thereof in a prior-art example.

### DESCRIPTION OF THE PREFERRED ENFORCEMENT FORM

Various embodiments of the invention will hereunder be described in detail with reference to the drawings.

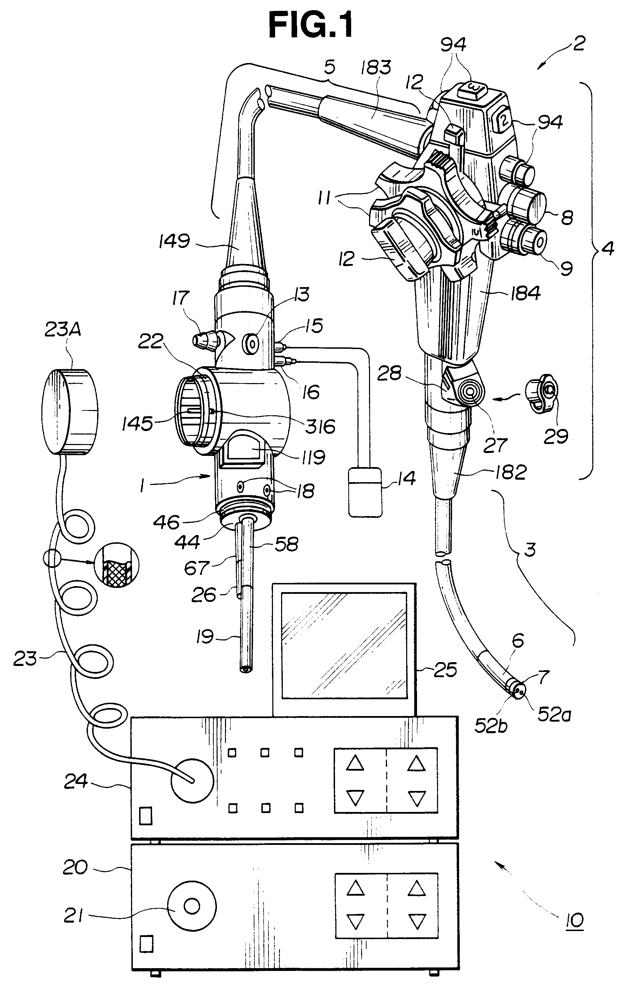

An endoscope apparatus 10 shown in Fig. 1 comprises an electronic endoscope 2 according to a first embodiment, a light source device 20 for supplying an illumination light, to which the electronic endoscope 2 is connected, a video processor 24 to which the electronic endoscope 2 is connected through a connection cord 23, for executing signal processing, and a color monitor 25 to which a video signal outputted from this video processor 24 is inputted, to thereby display a corresponding endoscope image.

The electronic endoscope 2 comprises an elongated insertion part 3 having elasticity, an operation part 4 thick in width formed at a rearward end of the insertion part 3, and a connection cord 5 having elasticity and extending from the operation part 4 (called also "universal cord"). The insertion part 3 is covered with elastic resin such as polyurethane or the like, and is provided, at a forward end thereof, with a forward-end hard part 7 which is formed by a hard member. A curvature part 6 which is covered with a soft resilient or elastic body is provided in the vicinity of the forward-end hard part 7.

The forward-end hard part 7 is provided with an illumination optical system 52a, an observation optical system 52b, a gas-supply and water-supply nozzle, a forceps-opening outlet and the like. Further, an unshown image pickup element provided with function to execute photoelectric conversion, such as a CCD or the like is provided at an imaging position of the observation optical system 52b. The forward-end hard part 7 is provided with one of openings of forceps channels which extend within the insertion part 3. A forceps opening cover 28 of the operation part 4 is provided with the other opening for the forceps channel, as a forceps opening 27. A forceps plug 29 is mounted, as occasion demands, on the forceps opening 27.

A line changeover device 8 which is used when suction is executed, a gas-supply and water-supply valve 9 which is operated when gas supply and water supply are executed, and further, remote switches 94 for processing the video signal which is outputted from the image pickup element are mounted to the operation part 4.

Moreover, angle knobs 11 made of hard resin are mounted on the operation part 4. These are operated whereby it is possible to curve the curvature part 6 in upward and downward directions and left- and right-hand directions. Furthermore, engaging levers 12 made of hard resin, which are used when the curvature part 6 is kept under a curved fixing state, or when a curved fixing state is released, are also mounted. A grip part 184 which is gripped by an operator is provided at a portion of the operation part 4 near the insertion part 3.

The connection cord 5 is covered with resin such as polyurethane or the like. A proximal end portion thereof which extends from the operation part 4 is provided with a failure prevention 183. Moreover, a connector for the endoscope (hereinafter simply referred to as "connector") 1 which is formed by hard resin is mounted on a forward end (or a distal end) of the connection code 5. A failure prevention 149 having failure prevention function is formed at a portion at which the rearward end of the connector 1 and the distal end of the connection code 5 are connected to each other.

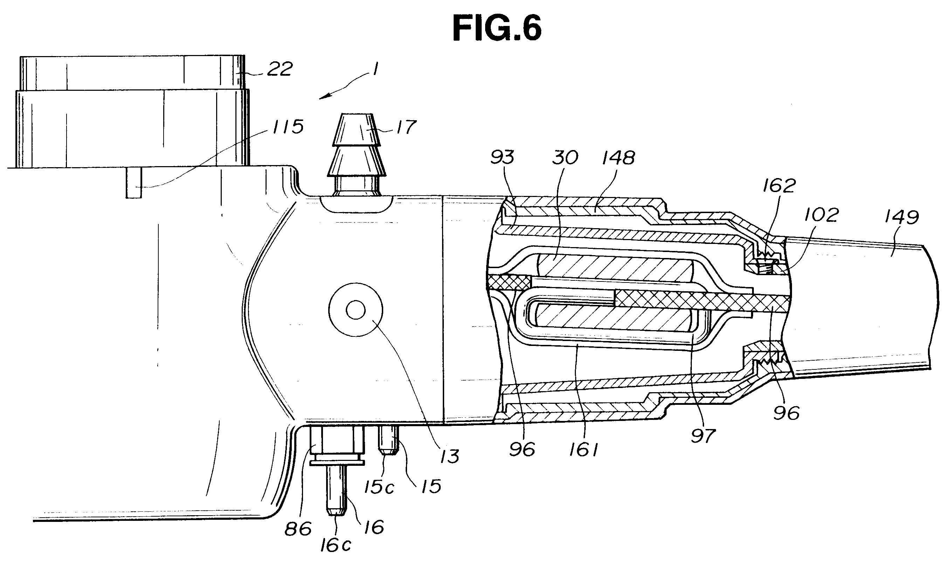

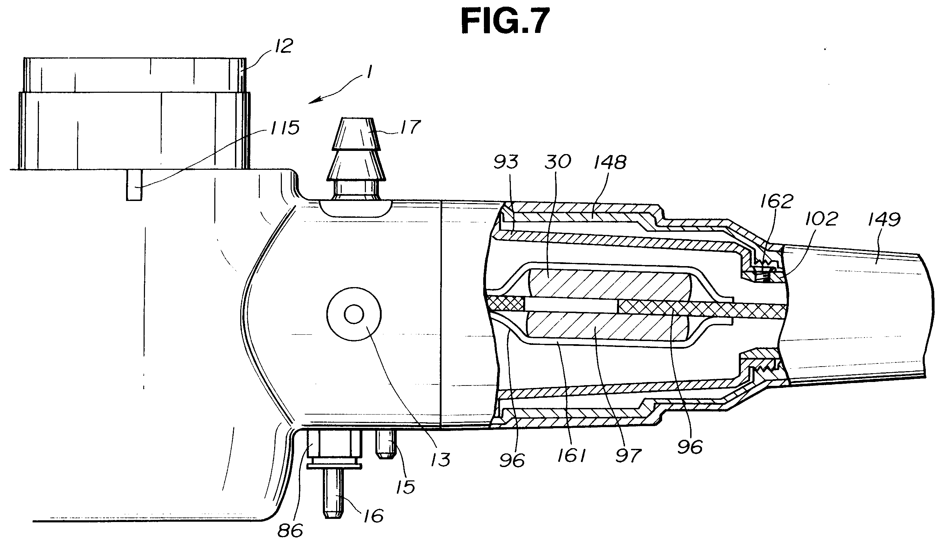

The embodiment is principally characterized in that a connection part 93 (refer to Fig. 6 and Fig. 7) which connects the rearward end of the connector 1 and the distal end of the connection cord 5 to each other is provided within the failure prevention 149, and a ferrite core 30 which serves as noise reduction means for reducing radiation of the noises from the signal cable and invasion of the noises to a signal cable 97, or the like, is provided within a connection part 93.

The connector 1 is provided with an earth terminal 13 for returning high-frequency leakage current to a cauterization power supply, a gas supply base 15 made of a metal which is connected to a water supply tank 14 for executing water supply, a water supply base 16, a suction base 17 connected to an unshown suction pump to execute suction, electric contacts 18 connected to the light source device 20, an end 19 of the light guide which is fixedly mounted to a light guide base 58 in the forward-end face thereof, and a fluid base 26 which is supported by a fluid-base support member 67.

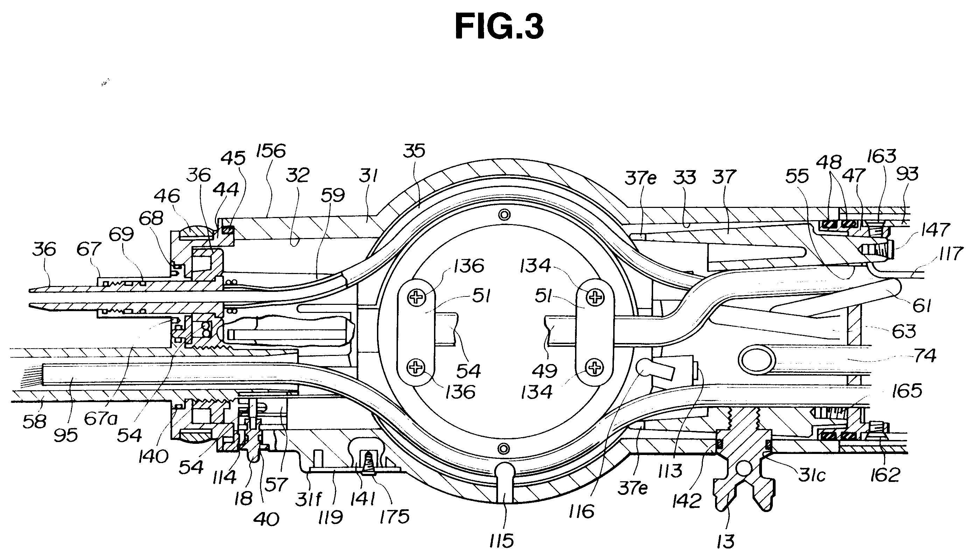

The end 19 of the light guide is connected to a connector receipt 21 of the light source device 20, whereby the illumination light is supplied to the end 19. The illumination light is transmitted by a light guide fiber 95 (refer to Fig. 3, for example) so that the illumination light is outputted from the illumination optical system 52a of the forward-end hard part 7. Thus, the side of a front object is illuminated. Moreover, pressurized air is sent to the fluid base 26 adjacent to the end 19 of the light guide, by a pump within the light source device 20.

Furthermore, an electric connector part 22 within which signal contacts 145 are provided in projection is provided on a side face of the connector 1. To this portion, a connector receipt 23A of the second connection cord or a second signal cable 23 is connected, and is connected to the video processor 24, whereby it is possible to electrically connect the image pickup element and the video processor 24 which serves as a signal processing device, to each other through the signal cable 97 (refer to Figs. 6, 7 and 8) within the electronic endoscope 2 and the second connection cord (signal cable) 23. The second connection cord 23 has a shielding member such as a metal mesh pipe or the like on the inside thereof which is covered with an insulating tube. On the inside thereof, a plurality of signal lines are inserted and pass.

A drive signal from an unshown drive-signal generation circuit within the video processor 24 is transmitted and is applied to the image pickup element to execute signal processing with respect to a photoelectrically converted image pickup signal which is outputted from the image pickup element, so as to be converted to the video signal to output the same to the color monitor 25. Thus, the arrangement is such that it is possible to display the endoscope image.

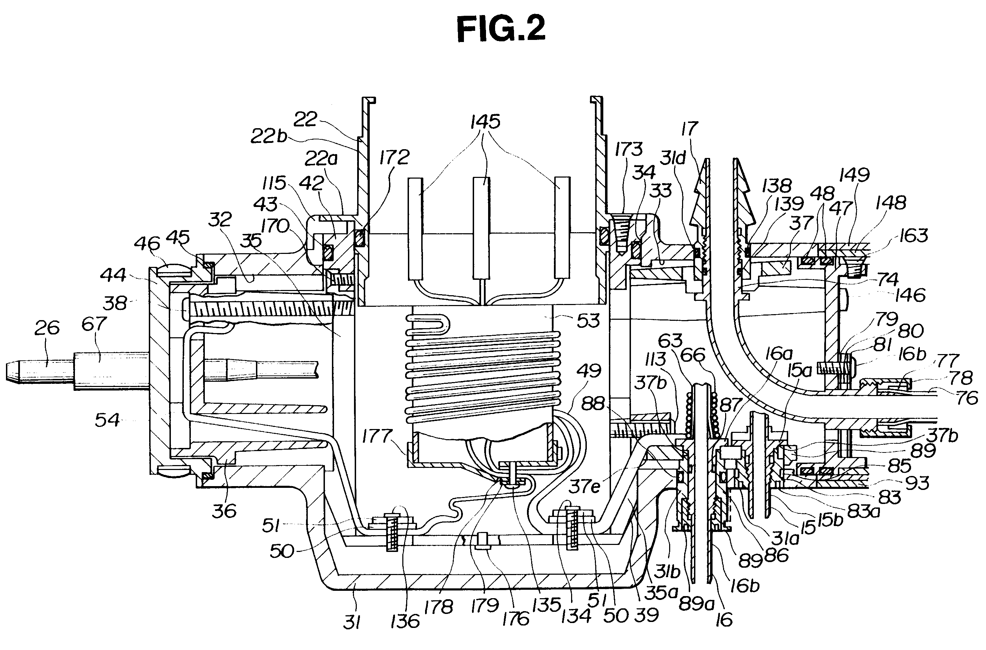

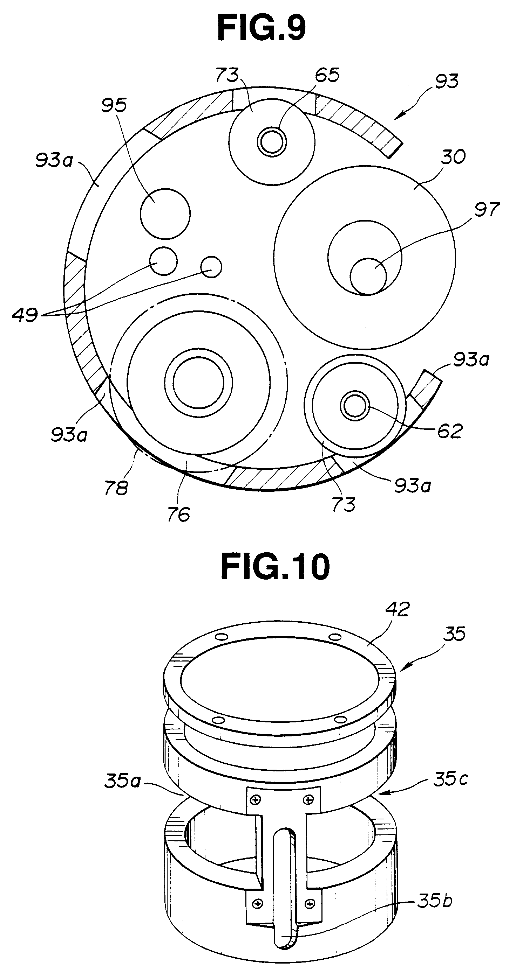

As shown in Figs. 2, 3 and 4, the connector 1 is provided with a connector case 31. Further, the connector case 31 is provided with a first opening 32 which opens toward the forward end of the connection code 5 so as to become an opening which is connected to the light source device 20, a second opening 33 which opens to a proximal end of the connection code 5 and which opens toward the side connected to the operation part 4, and a third opening 34 which opens toward the side which is connected to the video processor 24. The connector case 31 is formed by integral molding of resin, for example. The light guide fiber 95, electric wires 49, and a base 35 which is provided with an opening or a groove for inserting therethrough a tube or the like are mounted within the connector case 31. Form or contour of the base 35 is shown in Fig. 9.

As shown in Fig. 2, the electric connector part 22 has a proximal end thereof which is fitted and inserted through an opening in a base body 42 which is fixed to the connector case 31. A seat face 22a thereof is fixed by a screw 173. Furthermore, an O-ring 172 is interposed between the same and the base body 42 to form a watertight structure. A seal part 22b is formed in a side peripheral face which projects from the seat face 22a (toward the connector 1).

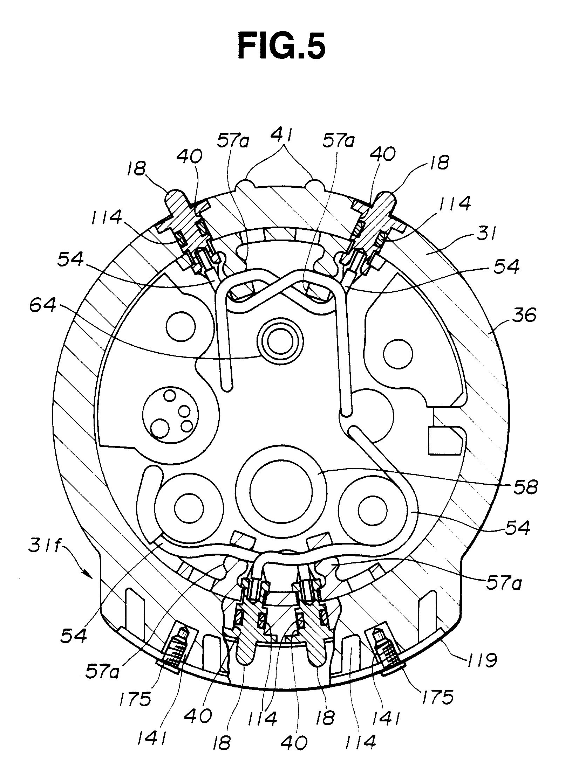

Further, as shown in Fig. 5, the connector case 31 in the vicinity of electric-contact fitting holes 40 into which electric contacts 18 are respectively fitted is provided with few projections 41 (two locations in the illustrated example). Moreover, a name plate 119 and a fixing part 31f also form projections. Furthermore, the name plate 119 is fixed to a name-plate fixing part 141 by a screw 175.

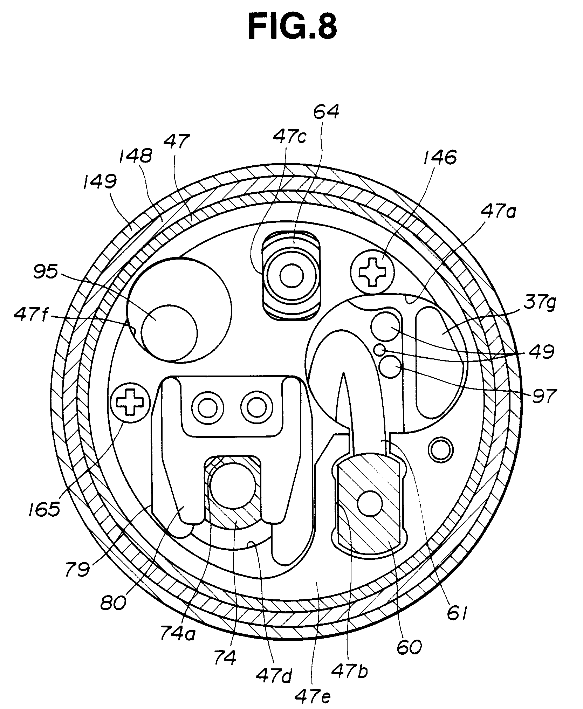

Fig. 6 is a cross-sectional view of the connection part 93 for connecting the connection cord 5 having the proximal end thereof which is connected to the operation part 4 of the electronic endoscope 2, and the connector 1 which is inserted into the light source device 20, to each other, and through which the signal cable is inserted and passes. In Fig. 8, a structure or construction of the connection part is shown in transverse cross-section.

The failure prevention 149 at the rearward end of the connector 1 is provided, on an inner side thereof, with a failure-prevention insert metal 148. The connection part (or a connection-part body) 93 generally in the form of a cylinder is housed within the failure-prevention insert metal 148, and both ends thereof are fixed. That is, the end on the side of the connection code is fixed to a connection-cord base 102 by a screw 162. As shown in Fig. 2, an end on the connector is fixed to a base fixing plate 47 by a screw 163.

The ferrite core 30 which has a cylindrical form and which is thick in wall, through which the signal cable 97 covered with a metal mesh pipe 96 is inserted and passes, is provided within the connection part 93. The ferrite core 30 which serves as an electromagnetic absorber which absorbs an electromagnetic wave is covered with a heat contraction tube 161, whereby the ferrite core 30 is fixed to the signal cable 97.

At this time, as shown in Fig. 6, the arrangement may be such that the signal cable 97 is looped around the ferrite core 30, and the signal cable 97 is made slightly longer than the connection cord 5. In a case where repair or the like is executed, if the signal cable 97 is shortened, the arrangement may be as shown in Fig. 7. Alternatively, the arrangement may be such that the ferrite core 30 passes under such a state as not to be made to the loop as shown in Fig. 7 from the beginning.

The signal cable 97 has one end thereof (on the side of the endoscope body) which passes through an electric-wire-signal-cable insertion and passage hole 47a in a base fixing plate 47, together with the image pickup element, and the other end which passes through the electric-wire-signal-cable insertion hole 47a in the base fixing plate 47, together with the electric wires 49 as shown in Fig. 8. The signal cable 97 is connected to the signal contacts 145 through an unshown printed circuit board within the electric connector part 22 (together with the electric wires 49).

Further, as shown in Fig. 9, the ferrite core 30 is arranged within the connection part 93 so as not to interfere with the electric wires 49 (which connect the remote switches 94 and the printed circuit board of the electric connector part 22 to each other), the gas supply tube 62, the liquid supply tube 65 and the light guide fiber 95. Moreover, the arrangement is such that the connection part 93 is provided with the windows 93a so that a space within the connection part 93 can be taken much.

As shown in Fig. 10, the base 35 is formed tubularly as viewed from the direction of the first opening 32, which serves as the light source device, and has an electric-wire groove 35a, an electric-wire groove 35b and a groove 35c.

A first body block 36 and a second body block 37 are inserted and pass respectively through the first opening 32 and a second opening 33 in Figs. 2, 3 and 4. The base body 42 is fixed to the base 35 by a screw 170, is fitted and inserted through the third opening 34, and is kept watertight by an O-ring 43. The first body block 36 is fixed to the base 35 by a fixing screw 38.

Furthermore, a C-shaped ring receipt 44 is water-tightly fitted and inserted through an opening end of the first opening 32 by an O-ring 45. Further, a C-shaped ring 46 is fitted over the C-shaped ring receipt 44. The connector 1 is adapted to be elastically engaged with the light source device 20 by the C-shaped ring 46.

As shown in Fig. 3, the second body block 37 is fixed to the base 35 by a screw 113, and is fixed to the second body block 37 by a screw 165 together with the base fixing plate 47 which is water-tightly fitted and inserted through an opening end of the second opening 33 by O-rings 48. The connector 1 is formed by the base 35, the first body block 36 and the second body block 37.

A discrimination or identification pin 115 (refer to Fig. 2, and Figs. 3, 6 and 7) and a waterproof-cap mounting index 116 (refer to Fig. 3) are fitted and inserted through a location between the base body 42 and the electric connector part 22. The identification pin 115 and the waterproof-cap mounting index 116 are fitted and inserted through a cut-out 31e which is formed in the connector case 31. Fixing of the identification pin 115 and the waterproof-cap mounting index 116 is also executed by the fact that the electric connector part 22 is threadedly engaged with the base body 42 by the screw 173.

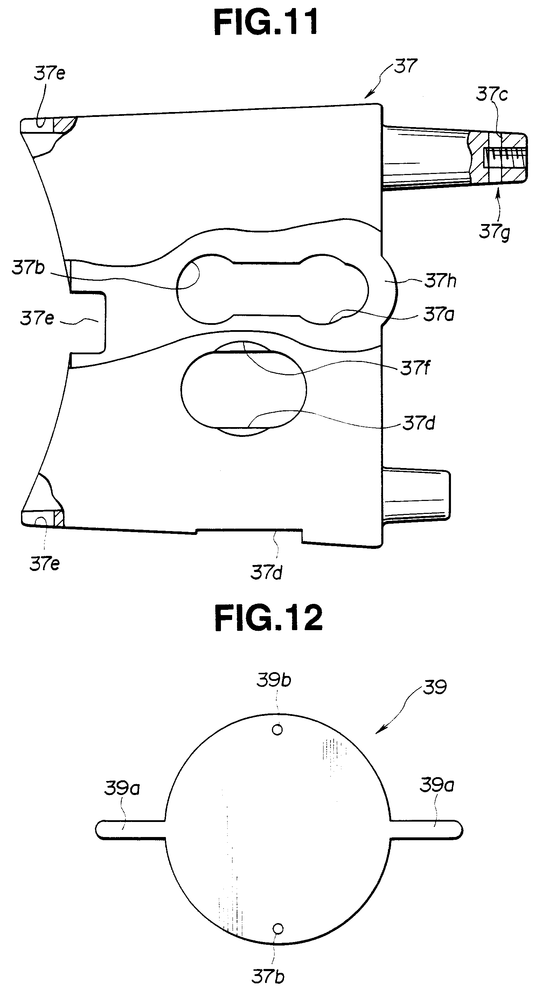

Further, the second body block 37 is so arranged as to wrap the base 35 and is made to the form shown in Fig. 11. Thus, recesses 37e are provided in the second body block 37 only at locations through which the electric wires 49, a fluid tube 59 and the light guide fiber 95 pass.

As shown in Fig. 11, in view of the strength and the molding, a gas supply base hole 37a and a water supply base hole 37b do not pass through the second body block 37 longitudinally.

Moreover, as shown in Fig. 2, on a bottom face of the base 35, a shielding plate 39 is provided to shield the bottom face. The shielding plate 39 shown in Fig. 12 has ears 39a which are bent. Thus, the shielding plate 39 is fixed to the bottom face of the base 35 by the screw 176 such that, as shown in Fig. 2, the electric-wire grooves 35a and 35b in the base 35 are also shielded.

Further, an unshown printed circuit board is stored within the electric connector part 22 which is fixed to the base body 42 shown in Fig. 2. The electric wires 49 and 54 and the signal cable 97 which are mounted in the connection cord 5 have forward ends thereof which are connected to the printed circuit board. As shown in Fig. 2, one of them, the electric wires 49, for example, has a length having a sufficient space. A halfway part thereof is engaged together by a screw. One of the electric wires 49 is engaged by a screw 134, while the other electric wire 54 is engaged by a screw 136. In that case, the electric wires 49 are urged and fixed by rubber plates 50 and presser plates 51.

A portion between the urging fixing part due to the screw 136 for the electric wire 54 and the urging fixing part which is provided on a shielding disk 177 due to a screw 135 is wound around a shielding tube 53 which is provided at a proximal end of the electric connector part 22. Moreover, a portion between the urging fixing part due to the screw 134 of the electric wires 49 and the urging fixing part due to the screw 135 of the shielding disk 177 is folded back and is stored in a space of the connector case 31. A rubber plate 178 for protection and a presser plate 179 are used also at the urging fixing part due to the screw 135.

Furthermore, the electric wire 54 is inserted and passes through the electric-wire groove 35b which is provided in the base 35 and, further, is inserted and passes through the first electric-wire insertion and passage hole 36a which is provided in the first body block 36. Moreover, the electric wires 49 are inserted and pass through the electric-wire groove part 35a which is provided in the base 35. The electric wires 49 pass through the second body block 37, are arranged at a second electric-wire insertion and passage hole 55 in the base fixing part 47, and are connected to the remote switches 94 which are provided on the operation part 4.

The signal cable 97 may also be inserted and pass or the like similarly to the electric wires 49 within the connector 1. Alternatively, the signal cable 97 may be separated from the electric wires 49 which are connected to the remote switches 94, within the connection part 93, and may pass through the ferrite core 30.

As shown in Fig. 3, the earth terminal 13 is water-tightly fitted and inserted through an earth terminal hole 31c in the connector case 31 by an O-ring 142, and is threadedly engaged with the second body block 37.

As shown in Fig. 5, the electric contacts 18 are fixed to the connector case 31 by a contact fixing plate (shown in Fig. 3) 57 which is provided on an inner peripheral face of the connector case 31. In this case, the electric contacts 18 are kept water-tight at a location between the same and the connector case 31 by O-rings 114. Further, sorting-out parts 57a of the electric wire 54 are provided on the contact fixing plate 57. The electric wire 54 is fitted into each of the sorting-out parts 57a, whereby the electric wire 54 is sorted out and held.

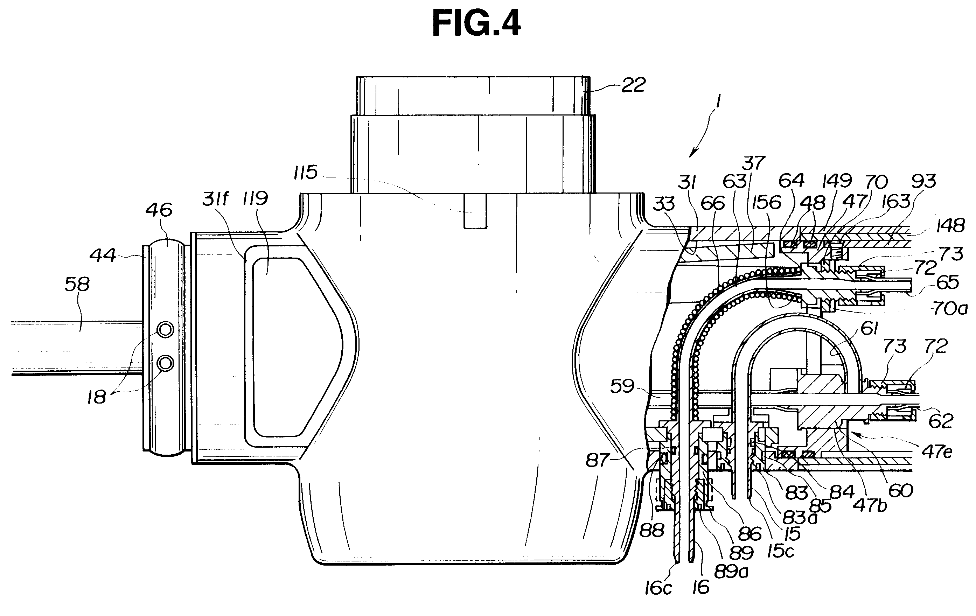

As shown in Fig. 4, a gas-supply pipe line system comprises the fluid base 26, the fluid tube 59, a gas supply port 60, a gas supply pipe 61, the gas supply base 15 and a gas supply tube 62. On one hand, a water-supply pipe line system comprises the water supply base 16, a water supply tube 63, a water supply port 64, and a liquid supply tube 65. Moreover, the water supply tube 63 is covered with a tightly wound coil 66. Furthermore, both of the gas supply tube 62 and the liquid supply tube 65 are connected to an unshown gas supply and water supply cylinder of the operation part 4.

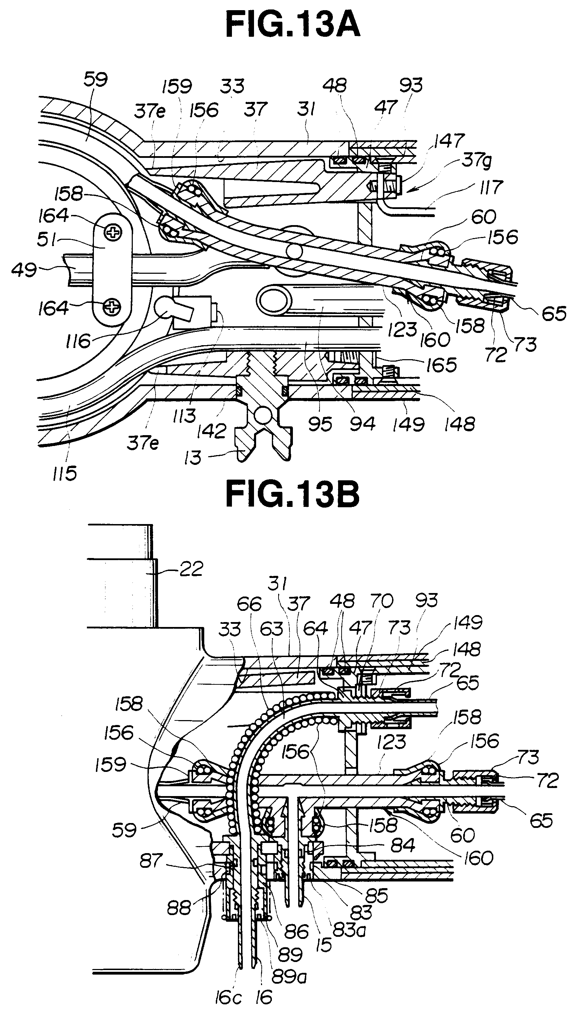

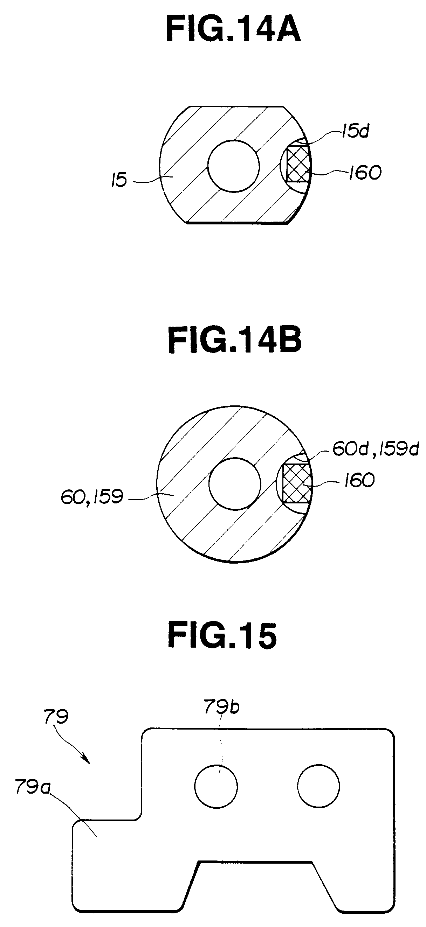

Further, as shown in Figs. 13A and 13B, a gas supply line 160 is formed by an elastic member 123. In this connection, since this elastic member 123 has directionality, the gas supply base 15, a side gas supply base 159 and the gas supply port 60 are provided respectively with cut-outs 15d, 159d and 60d as shown in Fig. 14A and 14B.

As shown in Figs. 3 and 4, the fluid tube 59 has one end thereof which is fitted into the fluid base 26 along the groove 35c in Fig. 10 and the other end which is connected to the gas supply port 60.

As shown in Fig. 3, the fluid base 26 is fitted into the first body block 36. The tubular fluid-base support member or tool 67 is threadedly engaged therewith and is fitted thereon. Further, a jig is mounted in a recess 67a (hereinafter referred to as "a pin face hole") for mounting an unshown jig which is provided on the fluid-base support member or tool 67, and the fluid-base support member or tool 67 is rotated, whereby the fluid base 26 and the C-shaped ring receipt 44 are fixed to the first body block 36.

In that case, the fluid-base support member or tool 67 and the C-shaped ring receipt 44 are kept water-tightly by an O-ring 68. Thus, the fluid base 26 and the fluid-base support member or tool 67 are kept water-tightly by an O-ring 69.

As shown in Fig. 3, the light guide base 58 is kept water-tightly by an O-ring 140, and is threadedly engaged with the first body block 36.

As shown in Fig. 4, the water supply port 64 is fixed to the base fixing plate 47 by a water-supply-port fixing nut 70. A jig is mounted to a groove 70a (hereinafter, referred to as "screw slot") to which a jig for rotating the water-supply-port fixing nut 70, which is provided on the water-supply-port fixing nut 70, can be mounted, and the water-supply-port fixing nut 70 is rotated whereby the water supply port 64 is fixed to the base fixing plate 47. The liquid supply tube 65 has an end thereof which is fitted in an end of the water supply port 64 on the operation part 4.

A tapered pipe 72 is fitted over the end of the liquid supply tube 65, and the tapered pipe 72 is tightened by a tapered tightening tube 73, whereby the liquid supply tube 65 is connected to the water supply port 64. Further, the water supply tube 63 is fitted in an end of the water supply port 64 on the electric connector part 22, and is fixed by a thread binding or tying adhesion 156.

Connection between the gas supply port 60 and the gas supply tube 62, connection between the fluid base 26 and the gas supply tube 62 and connection between the water supply tube 63 and the water supply base 16 are also similar to the above.

When the tapered tightening pipe 73 is tightened, the portion thereof forms a projection 47e such that a gas-supply port hole 47b is used also as a detent for the gas supply port 60.

As shown in Fig. 2, a suction line is formed by a single member (a suction pipe 74). As shown in Fig. 11, a suction-pipe mounting hole 37d in the second body block 37 forms an elliptic form. As shown in Fig. 2, the suction base 17 is threadedly engaged with the suction pipe 74.

Construction is such that water-tightness is held by an O-ring 139 at a location between the suction base 17 and the suction pipe 74, and a location between a suction base hole 31d in the connector case 31 and the suction base 17 is held in water-tightness by an O-ring 138.

A suction tube 76 which is connected to an unshown suction cylinder has one end face thereof which is fitted into an end face of the suction pipe 74 on the side of the operation part 4 and which is fixed by a tapered pipe 77 and a tapered tightening pipe 78.

Furthermore, a member which fixes the suction pipe 74 to the base fixing plate 47 is formed by an inflow prevention plate 79, a suction-pipe support plate 80 and a suction-pipe fixing plate 81.

Further, the inflow prevention plate 79 may be provided with a grip part 79a as shown in Fig. 15.

As shown in Fig. 2, the gas supply base 15 has a flange 15a and a forward-end opening 15b. The gas supply base 15 passes through a gas-supply base hole 31a in the connector case 31 and the gas supply base hole 37a in the second body block 37 under a state that a gas-supply-base fixing member 83 is threadedly engaged therewith, and the flange 15a is abutted against the second body block 37.

Moreover, a gas-supply-base fixing member 83 is water-tightly fitted over the forward-end opening 15b by an O-ring 84, and is held water-tightly by an O-ring 85 which is interposed between the same and a gas-supply base hole 31a in the connector case 31. A jig is mounted on a pin face 83a of the gas-supply-base fixing member 83, and the gas-supply-base fixing member 83 is rotated, whereby the gas supply base 15 is fixed to the connector case 31 and the second body block 37.

Furthermore, the water supply base 16 has a flange 16a and a forward-end opening 16b. The water supply base 16 passes through a water-supply base hole 31b in the connector case 31 and the water supply base hole 37b in the second body block 37, and the flange 16a is abutted against the second body block 37. Further, a base stopper 86 on the side of the water supply tank is water-tightly fitted and inserted through the water supply base 16 by an O-ring 87 and is water-tightly fitted and inserted through the connector case 31 by an O-ring 88,

Further, by the fact that a water-supply-base fixing nut 89 is threadedly engaged with the water supply base 16, the base stopper 86 on the water supply tank is fixed by the water-supply-base fixing nut 89. At this time, positioning of the water supply base 16 and the base stopper 86 on the water supply tank is executed by the water-supply-base fixing nut 89.

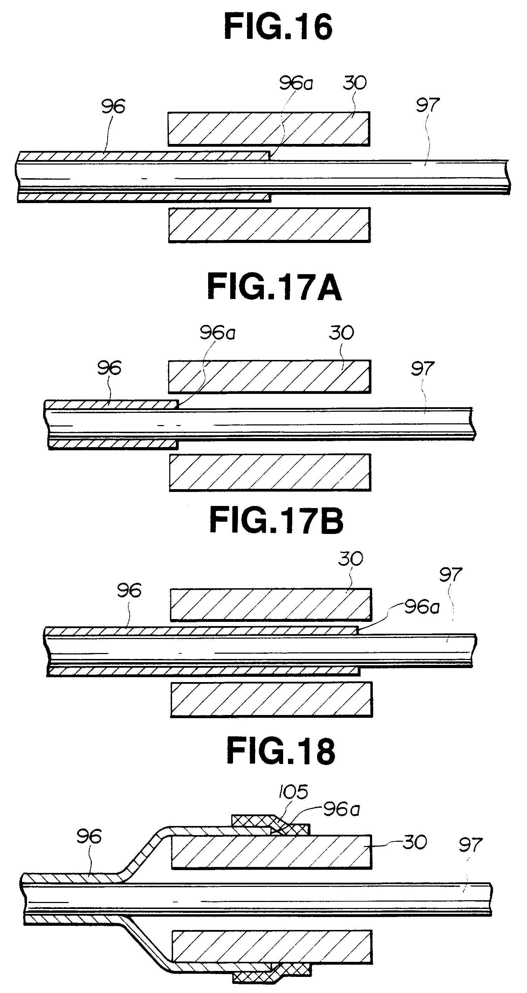

Further, as shown in Fig. 16, the signal cable 97 which covers the metal mesh pipe 96 is inserted and passes through the ferrite core 30. The metal mesh pipe 96 has the end part (one end face) 96a thereof which is so arranged as to be positioned within the ferrite core 30.

The invention achieves such the object as to provide the endoscope which enables the radiative noises from the signal cable 97 which is connected to the solid-state image pickup element which is provided within the forward end of the endoscope, to be reduced.

The construction which achieves the object may be one shown in Fig. 16, and may be a construction illustrated in Figs. 17A and 17B.

Specifically, the arrangement illustrated in Fig. 17A is such that the position of the end part 96a of the metal mesh pipe 96 within the ferrite core 30 is located in the vicinity of the end part of the ferrite core 30 at the grip of an operator as viewed from the insertion direction of the metal mesh pipe 96.

The arrangement illustrated in Fig. 17B is such that the position of the end part 96a of the metal mesh pipe 96 within the ferrite core 30 is located in the vicinity of the distal end part of the ferrite core 30 as viewed from the insertion direction of the metal mesh pipe 96.

Further, as shown in Fig. 18, the signal cable 97 which covers the metal mesh pipe 96 is inserted and passes through the ferrite core 30. The metal mesh pipe 96 covers also the ferrite core 30. Moreover, construction is such that the metal mesh pipe 96 has an end face 96a thereof which is fixed by a heat contraction tube 105.

Specifically, the arrangement may be such that the metal mesh pipe is covered over the signal cable, the ferrite core is covered with the metal mesh pipe which covers the signal cable, and the heat contraction tube is contracted to fix an end of the metal mesh pipe on the side of the ferrite core covering.

The object has been achieved which provides the electronic endoscope which enables the noises to be reduced, which are radiated or the like through the signal cable which is connected to the solid-state image pickup element which is provided in the forward end of the endoscope.

An FEP, a TFE, a PFA, silicon rubber or the like may be employed as a material of the heat contraction tube 105.

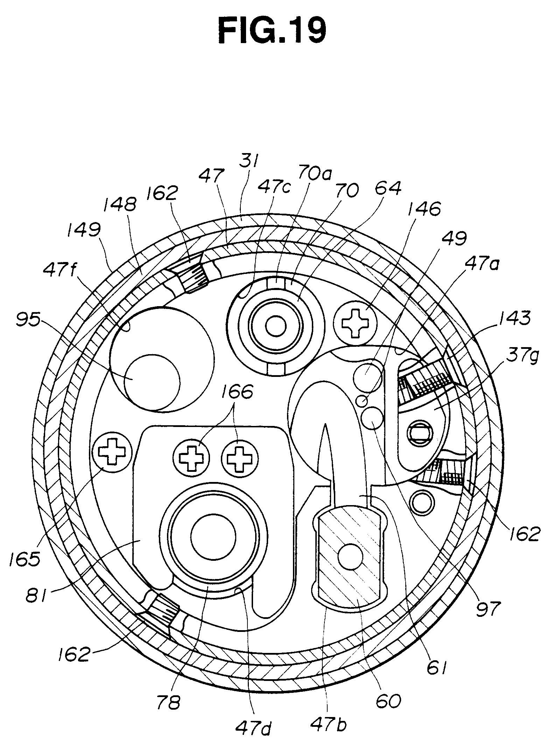

As shown in Figs. 8 and 19, the base fixing plate 47 is provided with the electric-wire-signal-cable insertion and passage hole 47a, the gas-supply port hole 47b, a water-supply port hole 47c, a suction pipe hole 47d and a light-guide-fiber insertion and passage hole 47f, which serve as boring holes which cause the electric wires 49 and the signal cable 97, the gas supply port 60, the water supply port 64, the suction pipe 74 and the light guide cable 95 to pass therethrough.

Subsequently, operation or function due to the arrangement of the first embodiment will hereunder be described.

As shown in Fig. 6 or 7, the ferrite core 30 through which the signal cable 97 which covers the metal mesh pipe 96 is inserted and passes is provided in the connection part 93 which is formed at the rearward end of the connector 1 which is provided at the distal end of the connection cord 5. At this time, the signal cable 97 may be looped around the ferrite core 30.

With such arrangement, the arrangement is such that, in the connection part 93, a magnetic flux (in practice, an electromagnetic wave which becomes noises) around the signal cable 97 is concentrated and is absorbed by the ferrite core 30 to prevent generation of the noises. Accordingly, it is possible to more reliably execute function of electromagnetic shielding with respect to the vicinity of the connector 1 in which radiation and invasion of the noises are liable to occur, or a portion which is liable in that shielding becomes insufficient, like a loop of the signal cable 97.

Moreover, the ferrite core 30 for executing electromagnetic shielding to the connection part 93 at a portion of the connector 1 on the rearward end thereof is provided whereby it is possible to cause it to have electromagnetic shielding function without the fact that the connector 1 is large-sized more than a case where it is provided on the connector 1.

Moreover, in the present embodiment, in order to connect the signal cable 97 which is connected to the image pickup element which is arranged within the tip or the forward end of the insertion part 3, to the video processor 24 which executes signal processing with respect to an external image pickup element of the electronic endoscope 2, it is necessary that the connector receipt 23A of the second connection cord 23 is connected to the electric connector part 22 of the connector 1, and the electric connector at the other end of the second connection cord 23 is connected to the video processor 24.

Specifically, signal transmission means for connecting the image pickup element and the signal processing device to each other comprises two signal transmission members (specifically, the signal cable 97 and the connection cord 23). The connector 1 (the end 19 of the light guide) which is connected to the light source device 20 for supplying the illumination light to the light guide fiber is formed in the vicinity of the electric connector receipt 23 and the electric connector part 22 which connects the two signal transmission members to each other.

Since, in the electric connector part 22 and the electric connector receipt 23, respective contacts are exposed, the electromagnetic waves are liable to be radiated to the outside as the noises also under a state that these are connected to each other, and the light source device 20 is arranged in the vicinity thereof. For this reason, in the present embodiment, the ferrite core 30 which serves as an electromagnetic absorber which absorbs electromagnetic waves is arranged in the vicinity of the connector 1 to prevent the noises from being radiated to peripheral devices. Furthermore, radiation of the noises from the light source device 20 is also absorbed by the ferrite core 30 to reduce that the noises invade into the side of the signal cable 97.

In this case, even if the ferrite core 30 is arranged in the vicinity of the connector of the signal processing device, it is almost impossible to reduce the noises which are radiated toward the light source device 20 from the vicinity of the electric connector part 22 and the electric connector receipt 23.

In connection with the above, in a case where the ferrite core 30 is provided therein, it is preferable that the ferrite core is provided at a position which does not interfere with the visceral objects. Furthermore, the ferrite core 30 may be latched along the inner peripheral face of the connection part 93.

Subsequently, assembling procedure of the connector 1 will be described.

One end of the electric wire 54 is soldered to the electric contacts 18. The electric contacts 18 are fitted in the contact fitting holes 40. The electric contacts 18 are fixed to the connector case 31 by the contact fixing plate 57.

The base body 42 is fixed to the base 35 by the screw 170.

The shielding plate 39 is fixed to the bottom face of the base 35 by the screw 176. The base 35 is fitted in the third opening 34 in the connector case 31 under a state that the ear 39a is folded. The second body block 37 is fitted and inserted by the second opening 33. The base 35 and the second body block 37 are fixed to each other by the screw 113 prior to the fact that the visceral object is assembled thereto.

The other end of the electric wire 54 passes through the first body block 36. The other end of the electric wire 54 passes through the groove 35b for the electric wire. The other end of the electric wire 54 is urged and fixed by the rubber plates 50 and the presser plates 51. The other end of the electric wire 54 is connected to an unshown printed circuit board. Further, the rubber plates 50 and the presser plates 51 are fixed to the base 35 by the screws 134 and 136, respectively.

The signal cable 97 and the electric wires 49 having their respective one ends which are connected to the unshown image pickup element, and the remote switches 94 pass through the electric-wire signal-cable insertion and passage hole 47a, pass through the second body block 37, and are inserted and pass through the groove 35a for the electric wire. Subsequent operation is the same as that of a case of said electric wire 54.

The first body block 36 is fitted and inserted by the first opening 32, and is fixed to the base 35 by the fixing screw 38.

The water supply tube 63 has one end thereof which is fitted in the water supply base 16. The coil 66 is outwardly mounted on the water supply tube 63. The other end of the water supply tube 63 is fitted in the water supply port 64. The water supply base 16 is fitted and inserted through the water-supply base hole 31b and the water supply base hole 37b.

The gas supply base 15 and the water supply base 16 are assembled together. The fluid tube 59 has one end thereof which is fitted in the fluid base 26, and the other end which is fitted in the gas supply port 60. The gas supply base 15 is fitted in and is inserted through the gas-supply base hole 31a and the gas-supply base hole 37a.

As shown in Figs. 2 and 4, the gas supply base 15 and the water supply base 16 are abutted against the second body block 37 by their respective flanges 15a and 16a. The gas supply base 15 is fixed by the gas-supply-base fixing member or tool 83 and is water-tightly fixed to the connector case 31. The water supply base 16 and the base stopper 86 on the side of the water supply tank are fixed by the water-supply-base fixing member 89.

The suction pipe 74 is inserted and passes from the side of the second opening 33 toward the second body block 37, and a suction base 75 is threadedly engaged therewith. Adhesives are applied to the end face of the suction pipe 74 and the end face of the suction base 17.

The ring receipt 44 in the form of C is fitted over and is inserted through the connector case 31, and the fluid-base support member or tool 67 is threadedly engaged therewith. The light guide base 58 is threadedly engaged with the first body block 36.

The C-shaped ring 46 is mounted to the C-shaped ring receipt 44.

The earth terminal 13 is fitted in and is inserted through an earth terminal hole 31c, and is threadedly engaged with the second body block 37.

As shown in Fig. 19, the gas supply port 60, the water supply port 64 and the suction pipe 74 are arranged respectively at the gas supply port hole 47b, the water-supply port hole 47c and the suction pipe hole 47d, which are provided in boring at their respective predetermined positions of the base fixing plate 47, and are fitted in and inserted through the second opening 33.

The water supply port 64 is fixed to the base fixing plate 47 by the water-supply-port fixing nut 70.

The inflow prevention plate 79, the suction-pipe support plate 80 and the suction-pipe fixing plate 81 are arranged in order on the suction-pipe planar-face part 81, and are fixed by a screw 166.

Procedure to be connected to the operation part 4 is such that the water supply tube 63 and the liquid supply tube 65 are connected respectively to the gas supply port 60 and the water supply port 64 by the use of the tapered pipe 72 and the tapered tightening pipe 73.

At this time, in such a manner that the gas-supply port hole 47b serves also as a detent for the gas supply port 60, a portion thereof is made to the convex 47e.

Further, hitherto, an earth electric wire 117 has been soldered to the earth terminal 13. However, as shown in Figs. 3 and 11, the second body block 37 is provided with a hole 37c for inserting therethrough the earth electric wire 117. The earth electric wire 117 is inserted therethrough, and is urged and fixed by a screw 147.

Alternatively, it is also possible to solder a crimping terminal 118 to the earth electric wire 117 so that the crimping terminal 118 is fixed to the second body block 37 by the screw 147.

The connection part 93 is fixed to the base fixing plate 47 by the screw 163.

As shown in Fig. 19, a projection 37g of the second body block 37 and the connection part 93 are conducted to a screw 143.

The name plate 119 is fixed to the nameplate fixing part 31f of the connector case 31, by the screw 175.

The electric connector part 22 is fixed to the base body 42 by the screw 173. Further, the identification pin 115 (refer to Fig. 2, and Figs. 6 and 7) and the waterproof-cap mounting index 116 are fitted in and are inserted through the base body 42 at a location between the base body 42 and the electric connector part 22. These identification pin 115 and waterproof-cap mounting index 116 are fitted over and are inserted through the cut-out 31e which is formed in the connector case 31. Fixing of the identification pin 115 and the waterproof-cap mounting index 116 is also executed by threaded engagement of the electric connector part 22 to the base body 42 by the screw 173.

Further, an outer periphery of the signal cable 97 is covered with the metal mesh pipe 96, and is electromagnetically shielded.

As shown in Fig. 16, since the end face 96a of the metal mesh pipe 96 is positioned within the ferrite core 30, even if the noises leak from the end face 96a, the noises are absorbed by the ferrite core 30 so that the noises are prevented from leaking.

As shown in Fig. 17A, the position of one end face of the metal mesh pipe 96 within the ferrite core 30 may also be positioned in the vicinity of the hand end of the ferrite core as viewed from the insertion direction of the metal mesh pipe, or, as shown in Fig. 17B, the position of one end face of the metal mesh pipe 96 within the ferrite core 30 may be positioned in the vicinity of a distal end of the ferrite core as viewed from the insertion direction of the metal mesh pipe.

As described above, the outer periphery of the signal cable 97 is covered with the metal mesh pipe 96. As shown in Fig. 18, further, an outer peripheral face of the ferrite core 30 may be covered with the metal mesh pipe 96. The end 96a of the metal mesh pipe 96 may be fixed by the heat contraction tube 105.

As shown in Figs. 2 ∼ 4, the gas supply and water supply bases 15 and 16, the suction base 17 and the earth terminal 13 are assembled to the position where the gas supply and water supply bases 15 and 16, the suction base 17, and the base of the earth terminal 13 do not exist on the same cross-section, in a cross-section, in the longitudinal direction, of the axis of the connector for the endoscope, which electrically connects detachably the side of the endoscope body and the endoscope control device to each other and in a cross-section in a direction vertical or perpendicular to the longitudinal direction, making it possible to miniaturize the connector 1.

As shown in Figs. 2 ∼ 4, if the suction base, the gas supply base and/or the water supply base are assembled respectively at positions which are substantially opposed against each other, the connector 1 is not large-sized.

According to this first embodiment, the ferrite core 30 is provided in the connection part 93 between the connection cord 5 and the connector 1 without interfering with the other visceral objects, whereby it is possible to reduce the radiative noises through the signal cable 97 without the fact that the connector 1 is large-sized. In this connection, the arrangement may be such that a ferrite core which is separate from the ferrite core 30, or the like, passes also through the electric wires 49 which are separated from the signal cable 97, to reduce the radiative noises.

As shown in Figs. 3 and 5, the projections 41 are provided at a plurality of locations (two locations in the illustrated example) in the vicinity of the electric-contact fitting holes 40. When the connector 1 drops to a floor, since the projections 41 impinge against the floor prior to the fact that the electric contacts 18 impinge against the floor, it is possible to prevent the electric contacts 18 from getting out of order. Moreover, since the nameplate fixing part 31f becomes also a projection, it is possible to prevent the electric contacts 18 from getting out of order, similarly to the above.

As shown in Fig. 11, a suction-pipe mounting hole 37d in the second body block 37 is made elliptic, to improve the operability or workability.

By the fact that the member for fixing the suction pipe 74 to the base fixing plate 47 is formed by the inflow prevention plate 79, the suction-pipe support plate 80 and the suction-pipe fixing plate 81, it is prevented that the adhesives enter the inside of the connector case 31.

The adhesives are applied to the end faces of the suction pipe 74 and the suction base 75, whereby the arrangement is such that the operator is not hurt even if the operator touches the suction base by his finger.

Further, the suction-pipe support plate 80 is provided with a grip 80a as shown in Fig. 5, whereby it is made possible to finely regulate the suction-pipe support plate 80.

Moreover, hitherto, the earth electric wire 117 is soldered to the earth terminal 13. However, as shown in Figs. 3 and 11, the second body block 37 is provided with the hole 37c through which the earth electric wire 117 is inserted. The earth electric wire 117 is inserted therethrough, is urged and is fixed by the screw 147, whereby the workability is improved.

The arrangement can be made such that end faces 15c and 16c on the side of detachment between the gas supply base 15 and a base 92 of the water supply base 16 on the side of the water supply tank are rounded so that the base 92 on the side of the water supply tank is not shaved.

Furthermore, respective inner diameters of a gas supply line 106 and a water supply line 107 of the base 92 on the side of the water supply tank are made larger than the respective inner diameters of the gas supply base 15 and the water supply base 16, whereby cleaning ability of the base on the side of the water supply tank is improved.

The connection part 93 may be provided with the windows 93a so that it is possible to take a much space within the connection part 93.

The second body block 37 is inserted and is fitted from the second opening 33, and the base 35 and the second body block 37 are fixed to each other by the screw 113 prior to the fact that the visceral objects are assembled together. Accordingly, assembling ability is improved.

Moreover, since the second body block 37 is so arranged as to wrap the base 35, it is possible to prevent malfunction due to the noises from the peripheral equipment from occurring.

The gas supply base hole 37a and the water supply base hole 37b increase as shown in Fig. 11 so that the assembling ability is improved.

The procedure of connection to the operation part 4 is such that the water supply tube 63 and the liquid supply tube 65 are connected respectively to the gas supply port 60 and the water supply port 64 by the use of the tapered pipe 72 and the tapered tightening pipe 73. A grip space is provided in the gas supply port 60 and the water supply port 64, whereby it is possible to fix the gas supply port 60 and the water supply port 64.

The projection 37g on the second body block 37 and the connection part 93 are conducted to a screw 143, whereby it is possible to reduce the radiative noises. Since it is possible to prevent malfunction due to the noises from the peripheral equipment from occurring, it becomes possible to execute endoscope inspection and handling safely.

The name plate 119 is mounted on the plane the same as that of the connector case 31, whereby it is not ugly or unsightly in view of an outer appearance.

Furthermore, because of the arrangement as shown in Fig. 16, since the signal cable 97 is covered with the ferrite core 30 or the metal mesh pipe 96 over the whole or overall length thereof, it is possible to reduce the radiative noises from the signal cable 97, and it is possible to cut off the noises from the peripheral equipment.

In a case of the arrangement shown in Fig. 17A, since there are few metal-mesh-pipe covering parts of the signal cable 97 within the ferrite core 30, the radiative noises from the signal cable 97 are made easy to be absorbed into the ferrite core 30.

In a case of the arrangement shown in Fig. 17B, since there are many metal-mesh-pipe covering parts of the signal cable 97, the noises from the peripheral equipment are made easy to be cut off.

Furthermore, in a case of the arrangement shown in Fig. 18, since the signal cable 97 is covered with the ferrite core 30 or the metal mesh pipe 96 over the whole or overall length thereof, it is possible to reduce the radiative noises from the signal cable 97, and it is possible to cut off the noises from the peripheral equipment.

If the FEP, the TFE, the PFA, the silicon rubber or the like is adopted as the material of the heat contraction tube 105, the heat contraction tube which can be used is easy to be available since the heat contraction tube is a material which is used generally.

As shown in Figs. 1 to 4, the positional relationship of the gas supply base 15, the water supply base 16, the suction base 17 and the earth terminal 13, for example, which are assembled into the connector 1 for the endoscope is such that, on the cross-section of the axis of the connector 1 for the endoscope in the longitudinal direction and on the cross-section in the direction perpendicular to the longitudinal direction, since there exist respective no their bases, miniaturization of the connector 1 is made possible.

Furthermore, as shown in Fig. 2, since the suction base 17, and the gas supply base 15 and the water supply base 16 are provided in opposed relation to each other, the connector 1 is not large-sized.

Subsequently, a second embodiment of the present invention will be described.

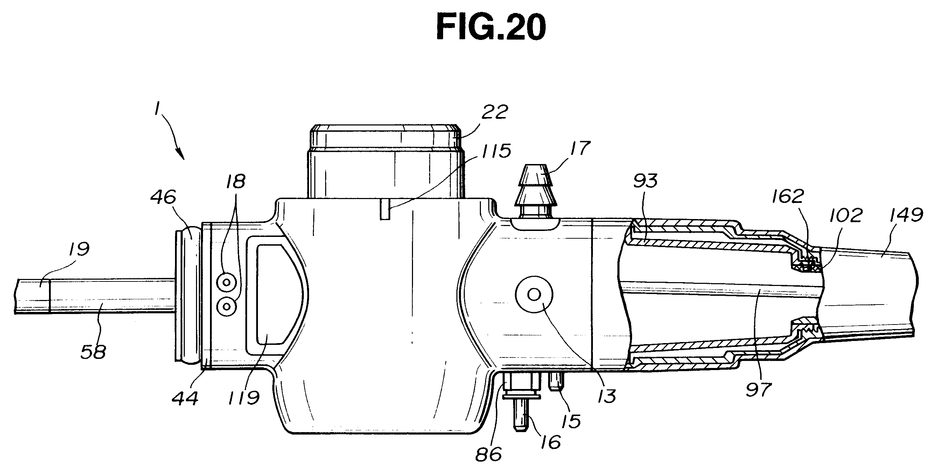

As shown in Fig. 20, the signal cable 97 which has one end thereof connected to the solid-state image pickup element is inserted and passes through the connection part 93 which connects the connection cord 5 having one end thereof which is connected to the operation part 4 of the endoscope and the connector 1 which is inserted into a socket of the light source device, to each other. The connection part 93 is formed by a ferrite material.

The connection part 93 is fixed to the base fixing plate 47 and the connection-code base 102 by the screw 162.

Subsequently, operation of this embodiment will be described.

The signal cable 97 is inserted and passes through the connection part 93 which is formed by the ferrite material, and the connection part 93 is fixed to the base fixing plate 47 and the connection-cord base 102 by the screw 162. Accordingly, the number of parts can be reduced, and it is not necessary to be anxious about the interference with the other visceral objects. Thus, it is possible to reduce the radiative noises from the signal cable 97.

Subsequently, a third embodiment of the present invention will be described.

An object of the embodiment is to provide the ferrite core into the endoscope without large-sizing of the connector. In order to achieve the object, an arrangement is such that the heat contraction tube is covered over the ferrite core through which the signal cable is inserted and passes, the signal cable is latched to the ferrite core, the ferrite core is arranged at a predetermined position where a window of the connection part opens, and the ferrite core is latched along an inner peripheral face of the connection part.

The background which is made in this manner will first be described.

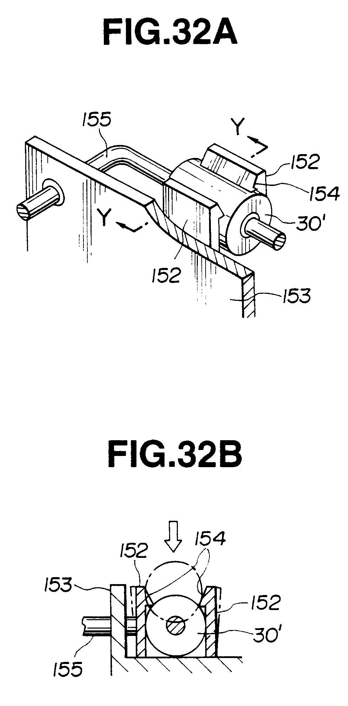

Hitherto, as disclosed in Japanese Patent Unexamined Publication No. HEI 2-308609 (308609/1990), fixing of a ferrite core 30' to a casing 153 has two opposed retaining or holding members 152 which are provided within the casing 153 of the input and output terminal device and supported resiliently or elastically in directions approaching each other, as shown in Fig. 32A, and the ferrite core 30' through which an interface cable 155 rendered from said casing 153 is inserted and passes.

The holding member 152 is provided with a pawl 154, having spring ability or elasticity such that the ferrite core 30' which is pressed into and is seized by the holding member 152 does not slip out or is not gotten away, so that the ferrite core 30' is seized and is held by the holding member 152. In this connection, Fig. 32B shows a Y - Y cross-section of Fig. 32A.

As is in this publication, in order that the ferrite core 30' is fixed to the casing 153, the ferrite core 30' has been pressed into, and been seized and been held by the pawl 154 which is caused to have spring ability.

However, in a case of this method, it is unreasonable that the ferrite core 30' is fixed to the hollow casing 153. If it can be fixed, the connector is large-sized.

Accordingly, in order to achieve the above-discussed object, the invention is made to an arrangement which will hereunder be described in detail.

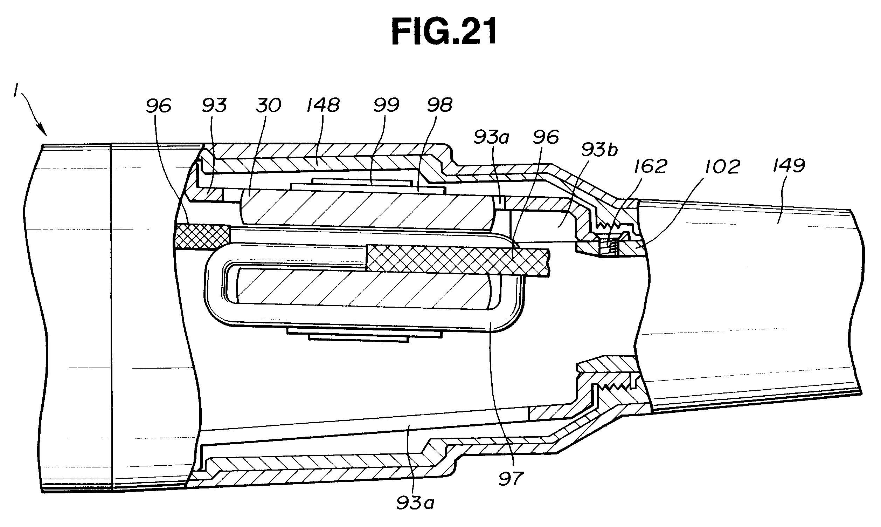

As shown in Fig. 21, the connection part 93 between the connection cord 5 having the one end thereof which is connected to the operation part 4 of the endoscope and the connector 1 which is inserted into the socket of the light source device is a hollow member and is provided with the windows 93a.

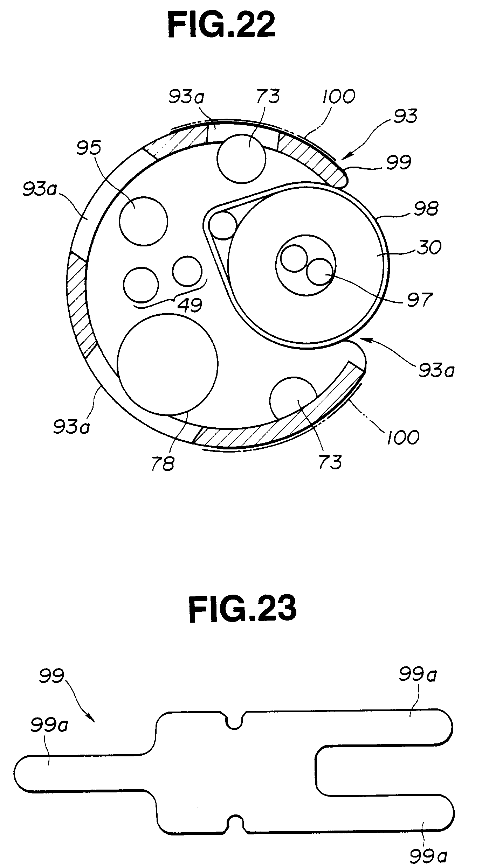

In an example in Fig. 22 which shows the cross-section of the connection part 93, the windows 93a are provided at four locations, and the ferrite core 30, the gas supply tube 62 (only the tapered tightening pipe 73 on the outside thereof is shown), the liquid supply tube 65 (only the tapered tightening pipe 73 on the outside thereof is shown), the light guide fiber 95, the electric wires 49 and the signal cable 97 are built therein.

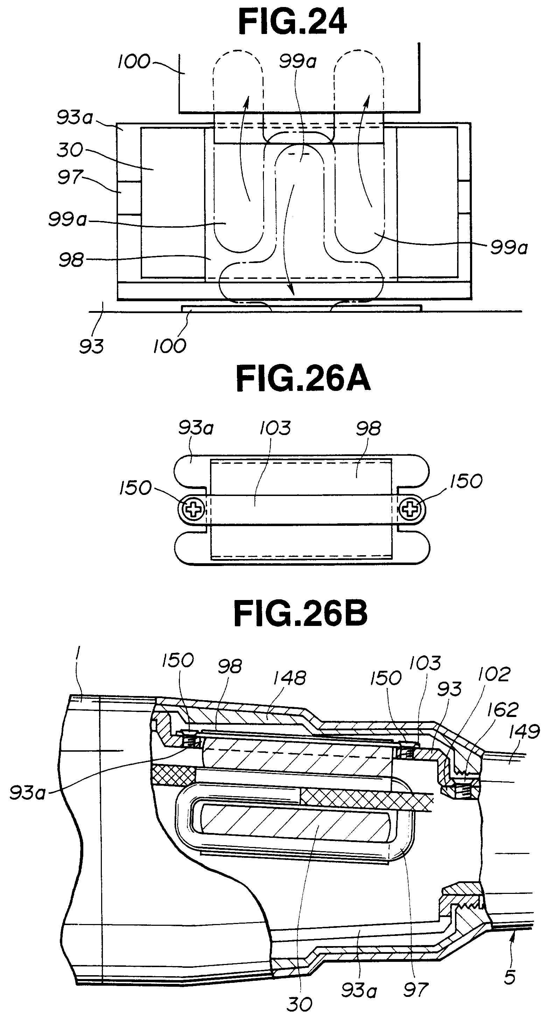

The signal cable 97 which covers thereon the metal mesh pipe 96 is inserted and passes through or is looped around the ferrite core 30. Further, a heat contraction tube 98 is covered on the ferrite core 30. Moreover, a thin metal plate 99 as shown in Fig. 23 is wound as shown in Fig. 24. An adhesive member such as an adhesive tape 100 or the like is pasted or put and fixed to an ear 99a of the metal plate 99.

Furthermore, a projection 93b inside the connection part 93 shown in Fig. 21 executes positioning of the ferrite core 30. Further, the connection part 93 is fixed to the connection-code base 102 or the base fixing plate 47 by the screw 162 or the like.

Although it has been described that the metal plate 99 is fixed by the adhesive member such as the adhesive tape 100 or the like, the metal plate 99 may be fixed in the following manner.

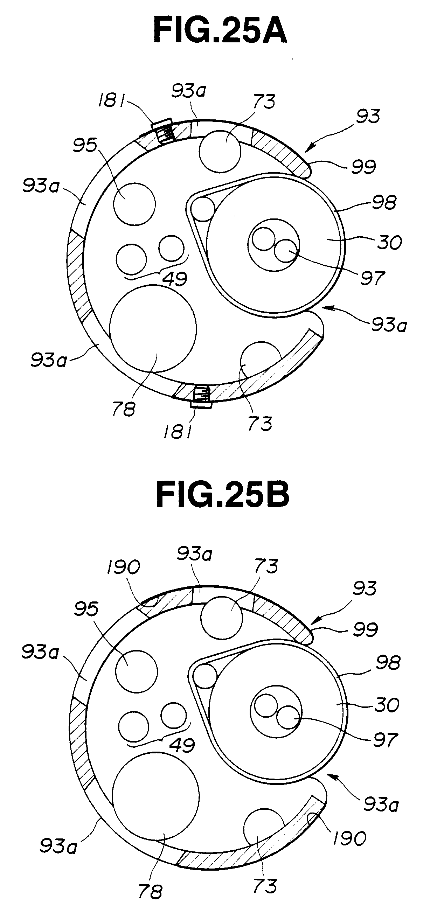

As shown in Fig. 25A, the metal plate 99 is fixed to the connection part 93 by a screw 181.

Alternatively, as shown in Fig. 25B, the metal plate 99 may be fixed to the connection part 93 by adhesives 190.

Subsequently, operation of this embodiment will be described.

The signal cable 97 which covers the metal mesh pipe 96 is inserted and passes through the ferrite core 30 and is looped at a determined position. The heat contraction tube 98 is covered around the ferrite core 30. The metal plate 99 as shown in Fig. 21 is covered around the ferrite core 30, and the connection part 93 is mounted.

The ferrite core 30 around which the metal plate 99 is covered is positioned at the predetermined windows 93a in the connection part 93. The ear 99a is folded along the outer peripheral face of the connection part 93. The adhesive member such as the adhesive tape 100 or the like may be pasted to the folded portion to fix the ferrite core 30.

Alternatively, the metal plate 99 may be fixed to the connection part 93 by the screw 181 as shown in Fig. 25A, or the metal plate 99 may be fixed to the connection part 93 by the adhesives 190 as shown in Fig. 25B.

With such arrangement, it is possible to position it to the connection part 93 between the connection cord 5 and the connector 1, without interference with visceral objects such as the electric wires 49, the gas supply tube 62, the liquid supply tube 65, the light guide fiber 95 and the like. Thus, it is made possible to reduce in diameter the connection part 93.

Moreover, the windows 93a open in the connection part 93. A space within the connection part 93 can be taken much without the fact that the connection part 93 is large-sized. Thus, it is possible to avoid interference of the visceral objects.

In the above-described arrangement, the signal cable 97 is latched (or fixed) to the ferrite core 30. The metal plate 99 is covered on the outer periphery thereof. The ferrite core 30 around which the metal plate 99 is covered is arranged at a predetermined position where the windows 93a in the connection part 93 open. The metal plate 99 is folded back so as to go along the outer peripheral face of the connection part 93, and is latched to the connection part 93. Accordingly, it is made possible to effectively utilize the space within the connection part 93.

In a case of being latched, the metal plate 99 is latched to an inner surface of the connection part 93 by the adhesive member, whereby it is possible to latch the ferrite core 30 by simple operation.

Alternatively, the metal plate 99 is latched to an inner surface of the connection part by the screw 181, whereby it is possible to latch the ferrite core 30 without executing special or specific operation.

Alternatively, the ferrite core 30 is latched along the outer periphery of the connection part 93 by the adhesives 190, whereby it is possible to reduce the number of parts.

Alternatively, the thickness of the above-described metal plate 99 is made equal to or less than 0.1, and the metal plate 99 is made to go along the outer peripheral face of the connection part 93, whereby, since the metal plate 99 is thin in thickness, the ferrite core 30 which covers the heat contraction tube 98 and the metal plate 99 is not large-sized. Thus, it is possible to be prevented that the ferrite core 30 interferes with other visceral objects.

Further, the material of the heat contraction tube 98 is made to the FEP, the TFE, the PFA, the silicon rubber or the like, whereby it is possible to use a heat contraction tube of a general material.

Moreover, the adhesive member is made to the adhesive tape 100, whereby it is possible to latch the ferrite core 30 by simple operation.

Furthermore, the adhesives 190 are made to an urethane system, an epoxy system, an acrylic system or the like, whereby it is possible to latch the ferrite core 30 by adhesives which are easy in purchasing.

Subsequently, a forth embodiment of the present invention will be described. The embodiment has also an object to provide an electronic endoscope which can be provided within an endoscope without the fact that the connector is large-sized similarly to the third embodiment. A metal plate is interposed, between the heat contraction tube and the ferrite core, in the ferrite core through which the signal cable is inserted and passes, and the heat contraction tube is shrunk. The metal plate is latched to the connection part, whereby the arrangement is such that the ferrite core is latched. Thus, the above-described object is achieved.

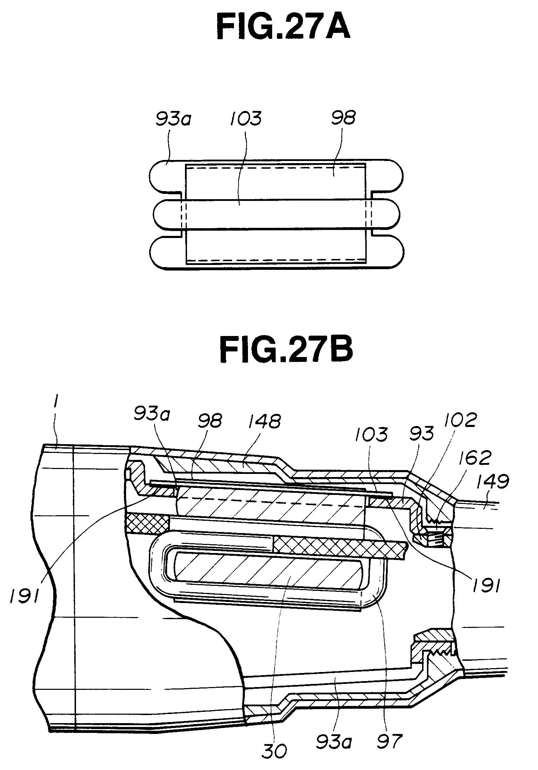

The embodiment will hereunder by described in detail with reference to Figs. 26A ∼ 29B. Figs. 26A, 27A and 28A show latching means portions in a case of being seen respectively from the above sides of Figs. 26B, 27B and 28B, which are anchored or latched respectively to the inner surface of the connection part by a screw 150, the adhesives 190 and the adhesive member.

As shown in Fig. 26B, the connection part 93 between the connection cord 5 (which has one end thereof which is connected to the operation part 4 of the electronic endoscope) and the connector 1 (which is inserted or thrust into the socket of the light source device) is a hollow member, and the windows 93a open at four locations, for example. As shown in Fig. 9 or 22, the ferrite core 30, the gas supply tube 62 (there is also the gas supply tube 62 which is shown only by the tapered tightening pipe 73 on the outside thereof), the liquid supply tube 65 (there is also the liquid supply tube 65 which is shown only by the tapered tightening pipe 73 on the outside thereof), the light guide fiber 95, the electric wires 49 and the signal cable 97 are incorporated in the connection part 93.

The signal cable 97 which covers the metal mesh pipe 96 is inserted and passes through or is looped around the ferrite core 30. Further, the heat contraction tube 98 is covered on the ferrite core 30, and a metal plate 103 which is punched at two locations is interposed therebetween. The heat contraction tube is fixed to the connection part 93 by the screw 150 at the position of each hole in the metal plate 103.

Moreover, the connection part 93 is fixed to the connection-code base 102 and the base fixing plate 47 by the screw 162 or the like.

In Fig. 26B, the metal plate 103 is fixed by the screw 150. In place thereof, however, as shown in Figs. 27A and 27B, the metal plate 103 may be fixed to the connection part 93 by adhesives 191 (which are applied to a face of the metal plate 103 in the vicinity of both ends thereof on the fixing side thereof). This is only different in the latching means, but the others are a similar arrangement as that in Fig. 26B.

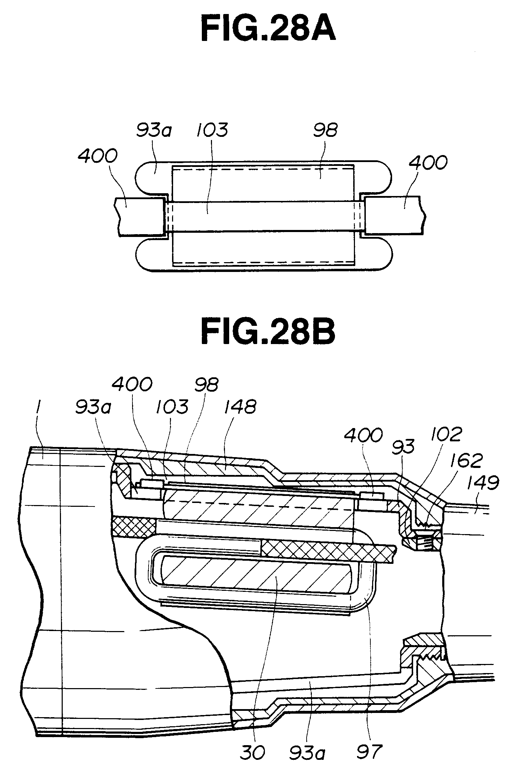

Moreover, in place of the fact that the metal plate 103 is fixed to the connection part 93 by the adhesives 191 as shown in Figs. 27A and 27B, the metal plate 103 may also be fixed to the connection part 93 by an adhesive member such as an adhesive tape 400 or the like as shown in Figs. 28A and 28B.

Subsequently, operation of the embodiment will be described.

The signal cable 97 which covers the metal mesh pipe 96 on the ferrite core 30 is inserted and passes and is looped at the determined position. The heat contraction tube 98 is shrunk through the metal plate 103 to a location between the same and the ferrite core 30. The connection part 93 is mounted, and the ferrite core 30 is exposed from the windows 93a. The ferrite core 30 can be fixed to the connection part 93 by the latching means.

According to this embodiment, it is possible to position the same to the connection part 93 between the connection cord 5 and the connector 1 without interference with the visceral objects such as the electric wires 49, the gas supply and gas supply tube 62, the liquid supply tube 65, the light guide fiber 95 and the like, making it possible to reduce in diameter the connection part 93.

The windows 93a open at the connection part 93. It is possible that the connection part 93 is not large-sized, that the space within the connection part 93 is taken much, and that interference of the visceral objects is avoided.

As the latching means, the screw 150 as shown in Fig. 26 may be used. In this case, it is possible to latch the ferrite core 30 without execution of special or specific operation.

As shown in Figs. 27A and 27B, as the latching means, the metal plate 103 can also be fixed to the connection part 93 by the adhesives 191. In this case, it is possible to reduce the number of parts. Further, as the latching means, as shown in Figs. 28A and 28B, it is also possible to fix the metal plate 103 by an adhesive member such as the adhesive tape 400 or the like. In this case, it is possible to latch the ferrite core 30 by simple operation.

Moreover, the material of the heat contraction tube 98 is made to the FEP, the TFE, the PFA, the silicone rubber or the like, whereby it is possible to use a heat contraction tube of a general material.

Furthermore, the adhesive member is made to the adhesive tape 400, whereby it is possible to latch the ferrite core 30 by simple operation.

Further, the above-described adhesives are made to the urethane system, the epoxy system, the acrylic system or the like, whereby it is possible to latch the ferrite core 30 by adhesives which are easy in purchasing.

Subsequently, a fifth embodiment of the present invention will be described. This embodiment also has an object to provide a connector which is capable of executing small-sizing or miniaturization similarly to the third embodiment. The arrangement is such that the heat contraction tube is covered on the ferrite core which is inserted and passes through the signal cable, the metal plate provided with a through hole is covered on the outer periphery of the ferrite core, the ferrite core around which the metal plate is wound is arranged at a position where the window in the connection part opens generally on the cylinder, and the metal plate is folded back and is latched so as to go along the outer peripheral face of the above-described connection part.

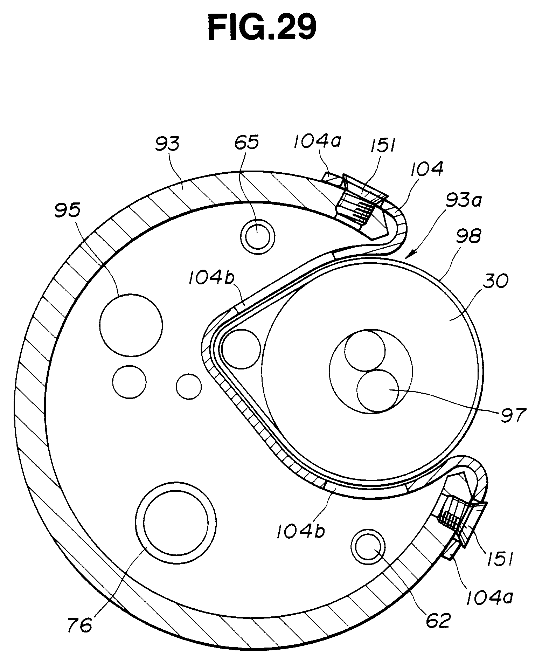

The embodiment will hereunder be described in detail with reference to Fig. 29 and the like.

As shown in Fig. 29, the connection part 93 between the connection code 5 having one end thereof which is connected to the operation part 4 of the endoscope, and the connector 1 which is inserted or thrust into the socket of the light source device is a hollow member. The windows 93a open at a single location in the illustrated example. The connection part 93 has incorporated therein the ferrite core 30, the gas supply tube 62, the liquid supply tube 65, the light guide fiber 95, the electric wires 49 and the signal cable 97.

The signal cable 97 (which is covered with the metal mesh pipe 96) is looped around the ferrite core 30. Further, the ferrite core 30 is covered with the heat contraction tube 98. Moreover, the heat contraction tube 98 is covered with a metal plate 104 which is provided therethrough a through hole 104b so as to be easy to bend the metal plate 104. The metal plate 104 is fixed to the connection part 93 by a screw 151 at the position of the ear 104a.

Furthermore, the connection part 93 is fixed to the connection-cord base 102 and the base fixing plate 47 by the screw 162 or the like, as described above.

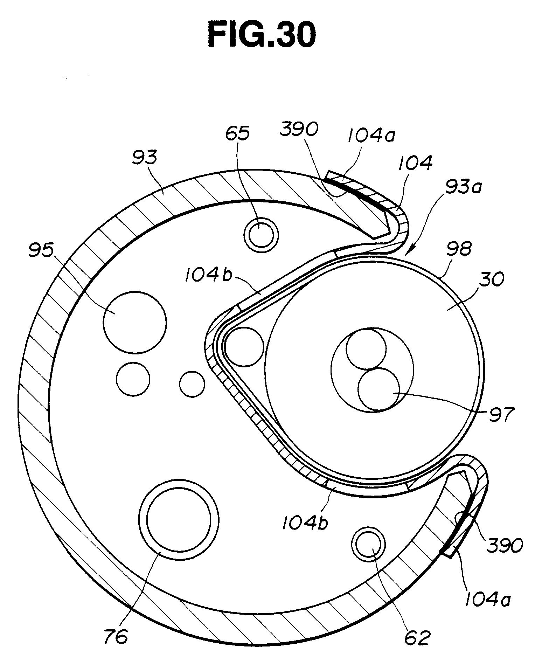

In Fig. 29, the screw 151 has been used as means for fixing or latching the metal plate 104 to the connection part 93. However, as shown in Fig. 30, the fixing may be executed by adhesives 390 which are applied to a face of the ear 104a on the fixing side, or the like.

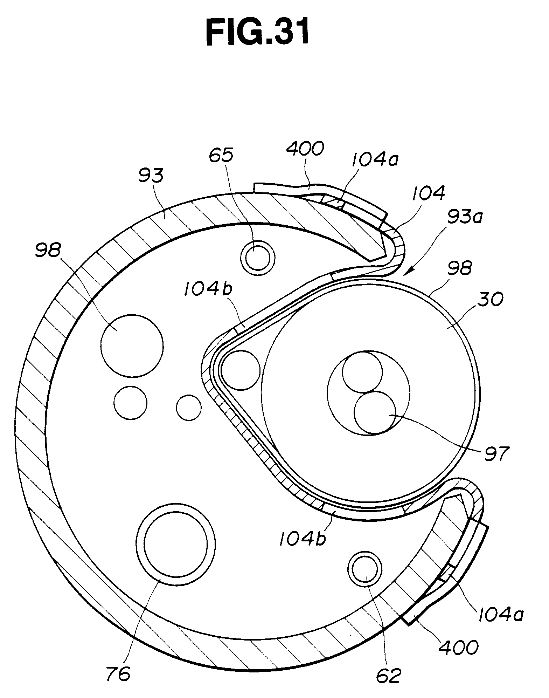

Moreover, as shown in Fig. 31, the metal plate 104 may be fixed by an adhesive member such as the adhesive tape 400 or the like.

Operation will subsequently be described.

The signal cable 97 which covers the metal mesh pipe 96 on the ferrite core 30 is inserted and passes and is looped at the determined position. The heat contraction tube 98 is covered on the ferrite core 30, and the heat contraction tube 98 is contracted or shrunk. Furthermore, the metal plate 104 is covered. The ferrite core 30 projects from the windows 93a, and is fixed to the connection part 93 by latching or fixing means such as the screw 151 or the like.

According to the embodiment, it is possible to position it to the connection part 93 between the connection pipe 5 and the connector 1 without interference with the visceral objects such as the electric wires 49, the gas supply and liquid supply tubes 62 and 65, the light guide fiber 95 and the like, making it possible to reduce in diameter the connection part 96.

The windows 93a open in the connection part 93. Thus, the connection part 93 is not large-sized, it is possible to take a much space within the connection part 93, and it is possible to avoid interference of the visceral objects.

It is possible to use the screw 151 as the engaging or latching means. In this case, it is possible to engage or latch the ferrite core 30 without execution of the specific or special operation.

Further, as the engaging or latching means, the metal plate 104 may be fixed to the connection part 93 by the adhesives 390 as shown in Fig. 30. In this case, it is possible to reduce the number of parts.

Moreover, as the engaging or latching means, the metal plate 104 may be fixed to the connection part 93 by an adhesive member such as the adhesive tape 400 or the like as shown in Fig. 31. In this case, it is possible to latch the ferrite core 30 by simple operation.

Furthermore, the FEP, the TFE, the PFA, the silicon rubber or the like is employed as the heat contraction tube 98, whereby it is possible to use a heat contraction tube of a material which is generally easy to be available.

Further, the urethane, the epoxy, or the acrylic systems is used as the adhesives 390, whereby it is possible to latch the ferrite core 30 by adhesives which are easy in purchasing.

Moreover, the adhesive tape 400 is employed as the adhesive member, whereby it is possible to latch the ferrite core 30 by simple operation.

As described above, according to the invention, in the electronic endoscope comprising the signal cable which is connected to the image pickup element, the connection cord through which the light guide fiber for transmitting the illumination light is inserted and passes, and which extends to the outside from the operation part, and the connector provided at the distal end of the connection cord and detachably connected to the light source device, the connection part which is provided in the vicinity of the distal end of the connection cord for connecting the connection cord and said connector to each other, and the electromagnetic absorber in which the signal cable is inserted through the connection part are provided therein. Accordingly, radiation of the noises from the signal cable to the peripheral devices such as the light source device and the like can be absorbed to restrain generation of the malfunction, and invasion of the noises into the signal cable from the peripheral devices can be prevented so that S/N of the image pickup signal can be improved.

In connection with the above, embodiments or the like which are arranged in partial combination or the like of the above-described embodiments or the like belong to the present invention.

## Claims

1. An electronic endoscope comprising an image pickup element arranged on the side of a forward end of an elongated insertion part and having function to execute photoelectric conversion, a signal cable which is inserted and passes through said insertion part, for transmitting a signal, and which is connected to the image pickup element, a light guide fiber inserted and passing through said insertion part, for transmitting the side of an illumination light, a connection cord extending from a rearward end of said insertion part, through which said signal cable and said light guide fiber are inserted and pass, and a connector provided at a distal end of said connection cord and detachably connected to a light source device for generating the illumination light,
wherein it comprises:
a connection part provided in the vicinity of the distal end of said connection cord and connected to said connector, and
an electromagnetic absorber arranged within said connection part and through which said signal cable passes, said electromagnetic absorber having function to absorb an electromagnetic wave.

2. An electronic endoscope according to claim 1, wherein said electromagnetic absorber is a ferrite core in the form of a cylinder.

3. An electronic endoscope according to claim 1, further comprising an electromagnetic shielding member for covering said signal cable to electromagnetically shield said signal cable.

4. An electronic endoscope according to claim 3, wherein said electromagnetic shielding member is a mesh pipe made of a metal, for covering said signal cable.

5. An electronic endoscope according to claim 1, wherein said connector has an electric connector which is connected to the distal end of said signal cable, and wherein a second signal cable which is provided with an electric connector receipt connected to said electric connector is connected to a signal processing device which executes signal processing with respect to said image pickup element.

6. An electronic endoscope according to claim 1, wherein said electromagnetic absorber is arranged at a position which does not interfere with visceral objects within said connection part.

7. An electronic endoscope according to claim 1, wherein said electromagnetic absorber in the form of a cylinder is arranged along an inner peripheral face of said connection part.

8. An electronic endoscope according to claim 7, comprising fixing means for fixing said electromagnetic absorber in the form of a cylinder, to an inner peripheral face of said connection part.

9. An electronic endoscope according to claim 7, wherein said fixing means is provided with a heat contraction tube for covering the cylindrical electromagnetic absorber through which said signal cable is inserted and passes, and a metal plate for further covering said electromagnetic absorber which covers said heat contraction tube.

10. An electronic endoscope according to claim 9, comprising any one of adhesives, an adhesion member and a screw, which fixes said metal plate to an inner face of said connection part.

11. An electronic endoscope comprising an electronic endoscope body having an image pickup element arranged on the side of a forward end of an elongated insertion part and having function to execute photoelectric conversion, a signal cable which is inserted and passes through said insertion part and which is connected to the image pickup element, for transmitting a signal, a light guide fiber inserted and passing through said insertion part, for transmitting an illumination light, a connection cord extending from the side of a rearward end of said insertion part, through which said signal cable and said light guide fiber are inserted and pass, a connector provided at a distal end of said connection cord and detachably connected to a light source device for generating the illumination light and an electric connector provided on said connector and to which the distal end of said signal cable is connected, and
a second signal cable having one end thereof which is provided with an electric connector receipt which is detachably connected to said electric connector and the other end which is connected to a signal processing device for executing signal processing with respect to said image pickup element,
wherein said signal cable passes in the vicinity of said connector, and wherein an electromagnetic absorber is provided which has function to absorb an electromagnetic wave.

12. An electronic endoscope according to claim 11, wherein said electromagnetic absorber is provided in the vicinity of the distal end of said connection cord, and is arranged within a connection part which executes connection with respect to said connector.

13. An electronic endoscope according to claim 11, wherein said electromagnetic absorber is a cylindrical ferrite core.

14. An electronic endoscope according to claim 11, comprising an electromagnetic shielding member for covering substantially the whole of said signal cable and having function to execute electromagnetic shielding.
